# EUROPEAN PATENT APPLICATION

(11) **EP 2 106 816 A2**
(43) Date of publication of application: **07.10.2009**
(21) Application number: 09250935.5
(22) Date of filing: 30.03.2009
(51) Int. Cl.: A61M 5/142, H05K 5/06, G06F 1/16

(54) **Over-molded seal**

(30) Priority: 31.03.2008 US 41175 P; 25.04.2008 US 109642
(71) Applicant: Animas Corporation, West Chester, PA 19380 (US)
(72) Inventor: Sharifi, Bahram, Schwenksville, PA 19473 (US)
(74) Representative: Tunstall, Christopher Stephen

(57) **Abstract**

Described is a seal used in a drug delivery device. In one embodiment, a seal (100) is provided that includes a tongue (102), a groove (104) and a coating (106) formed on at least one surface of the tongue, the groove or both the tongue and the groove. In another embodiment, a drug delivery device is provided that includes a housing having a top portion and a bottom portion, a tongue formed on the periphery of one of the top portion or bottom portion and a groove formed on the periphery of one of the top portion or bottom portion and configured to receive the tongue, and a coating on the surface of at least one of the groove and the tongue that creates a hermetic seal between the top portion and the bottom portion when the tongue and the groove are in contact.

## Description

### FIELD OF THE INVENTION

The present invention relates, in general, to over-molded seals and, more particularly, to over-molded seals used in drug delivery devices.

### BACKGROUND OF THE INVENTION

The use of drug delivery devices for various types of drug therapy is becoming more common in that drug delivery devices are used to automatically infuse a liquid form of a drug to a patient. The drug is typically pumped into the patient via a catheter or other injection device. For example, diabetics can utilize external infusion therapy for delivering insulin using drug delivery devices worn on a belt or in a pocket. The insulin is usually delivered via a catheter with a percutaneous needle or cannula placed in the subcutaneous tissue. In addition, infusion therapy is becoming more important for the treatment and control of other medical conditions, such as pulmonary hypertension, HIV and cancer.

The housing of the drug delivery device must be water-tight so that the internal components are not damaged when the drug delivery device is exposed to moisture. Typically, the housing is permanently sealed to prevent moisture ingress and the seal must be destroyed to service the drug delivery device.

Therefore, applicants recognize a need for a hermetic seal between a top portion and a bottom portion of the housing of a drug delivery device in which the seal is not damaged when the device is serviced.

### BRIEF DESCRIPTION OF THE DRAWINGS

The novel features of the invention are set forth with particularity in the appended claims. A better understanding of the features and advantages of the present invention will be obtained by reference to the following detailed description that sets forth illustrative embodiments, in which the principles of the invention are utilized, and the accompanying drawings of which:

FIG. 1 is a cross-sectional view a drug delivery device that may use a seal according to exemplary embodiments of the present invention;

FIGs. 2A and 2B are cross-sectional close-up views of a seal used in a drug delivery device according to an exemplary embodiment of the present invention;

FIGs. 3A and 3B are cross-sectional close-up views of a seal used in a drug delivery device according to another exemplary embodiment of the present invention;

FIGs. 4A and 4B are cross-sectional close-up views of a seal used in a drug delivery device according to yet another exemplary embodiment of the present invention;

FIG. 5 is a perspective view of a drug delivery device that is suitable for use with embodiments of the present invention; and

FIG. 6 is a perspective view of a top portion and a bottom portion of a housing for a drug delivery device that is suitable for use with embodiments of the present invention.

### DETAILED DESCRIPTION OF ILLUSTRATIVE EMBODIMENTS OF THE INVENTION

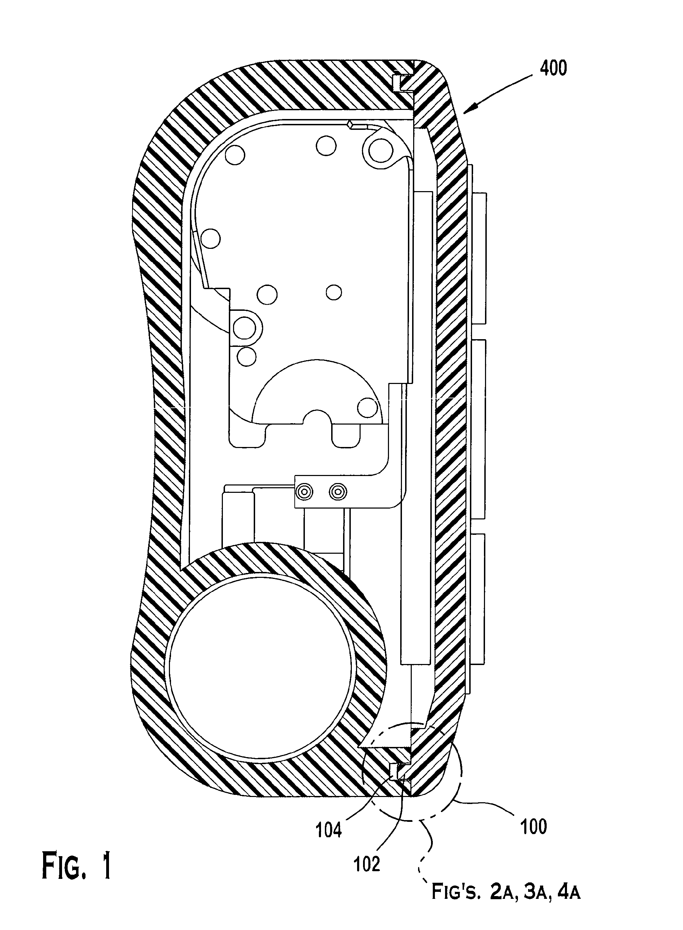

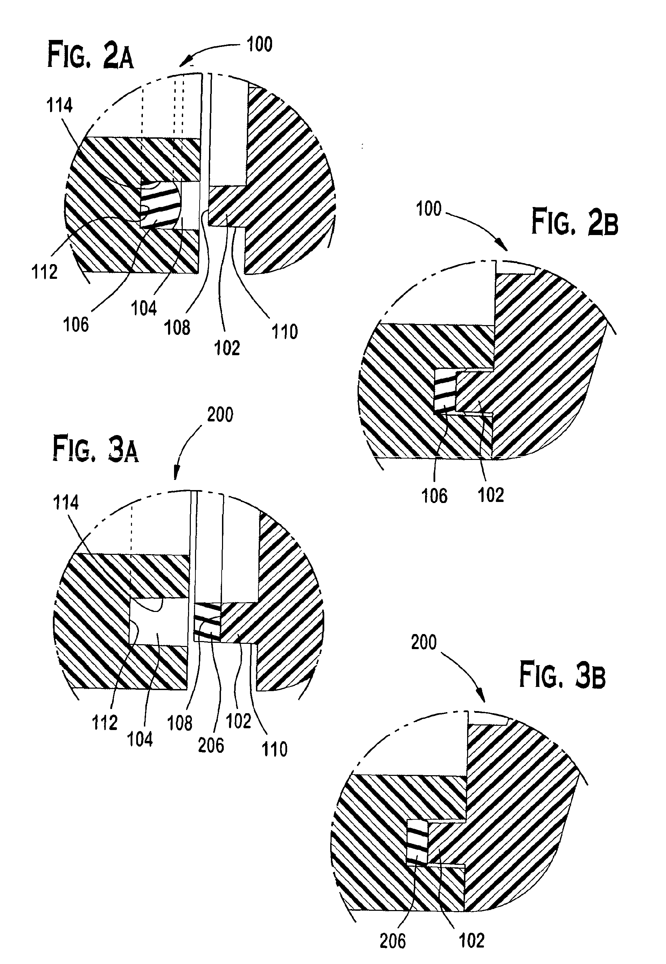

FIGs. 1, 2A and 2B illustrate a seal 100 according to an exemplary embodiment of the present invention. The seal 100 includes a tongue 102, a groove 104 and a coating 106. The tongue 102 includes a tip 108 and at least one side 110. The groove 104 includes a lower surface 112 and at least one wall 114.

The coating 106 may be over-molded onto at least one surface of the tongue 102 only, onto at least one surface of the groove 104 only or onto at least one surface of both the groove 104 and the tongue 102. The coating 106 shown in FIG. 2A covers the lower surface 112 of the groove 104 and fills substantially a third to a half of the volume of the groove 104. The coating 106 may, however, also be over-molded onto at least one wall 114 of the groove 104 such that the coating 106 covers the entire surface of the at least one wall 114 (not shown). As illustrated in FIG. 2B, when the tongue 102 is inserted into the groove 104, the coating 106 may be deformed such that a hermetic seal is formed around the tip 108 of the tongue 102 and at least partially up the at least one side 110 of the tongue 102.

In the exemplary embodiment of a seal 200 shown in FIGs. 3A and 3B, a coating 206 is over-molded onto at least one surface (e.g., the tip) of the tongue 102. The thickness of the coating 206 is such that when the tongue 102 is placed into the groove 104, the coating 206 fills substantially a third to a half of the volume of the groove 104. The coating 206 as shown in FIG. 3A is over-molded onto the tip 108 of the tongue 102 but may also be over-molded onto at least one side 110 of the tongue 102 such that the coating 206 covers the entire surface of the at least one side 110. As illustrated in FIG. 3B, when the tongue 102 is inserted into the groove 104, the coating 206 may be deformed such that a hermetic seal is formed around the tip 108 of the tongue 102 and at least partially up the at least one side 110 of the tongue 102.

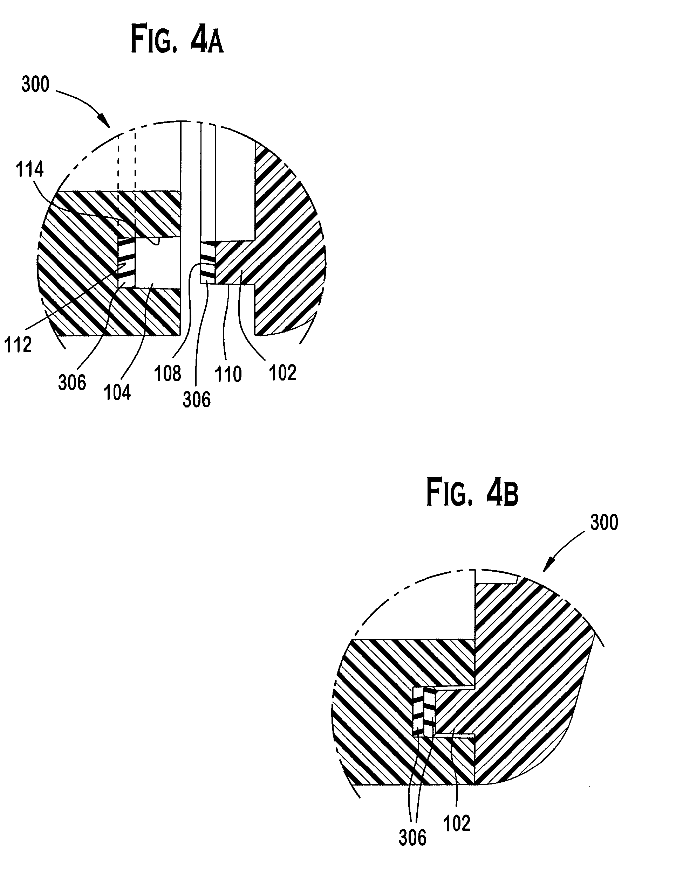

Another exemplary embodiment of a seal 300 is illustrated in FIGs. 4A and 4B in which a coating 306 is over-molded onto at least one surface (e.g., the lower surface 112) of the groove 104 and onto at least one surface (e.g., the tip 108) of the tongue 102. The total thickness of the coating 306 on both the groove 102 and the tongue 102 is such that when the tongue 102 is placed into the groove 104, the coating 306 fills substantially a third to a half of the volume of the groove 104. The coating 306 as shown in FIG. 4A is over-molded onto the tip 108 of the tongue 102 and onto the lower surface 112 of the groove 102 but may also be over-molded onto at least one wall 114 of the groove 104 and onto at least one side 110 of the tongue 102 such that all surfaces within the groove 104 and on the tongue 102 are completely covered with the coating 306 (not shown). As illustrated in FIG. 4B, when the tongue 102 is inserted into the groove 104, the coating 306 may be deformed such that a hermetic seal is formed around the tip 108 of the tongue 102 and at least partially up the at least one side 110 of the tongue 102.

The coatings 106, 206 and 306 may be formed of a thermoplastic elastomer having a hardness from about 35 Shore A to about 55 Shore A. Examples of coatings that may be used in the present invention include silicone rubber, a copolymer of butadiene and acrylonitrile, neoprene, Sorbothane^{®}, Santoprene^{®} or a combination thereof. The coatings 106, 206 and 306 may also be combined or over-coated with a material such as, for example, polyurethane to increase flexibility (not shown).

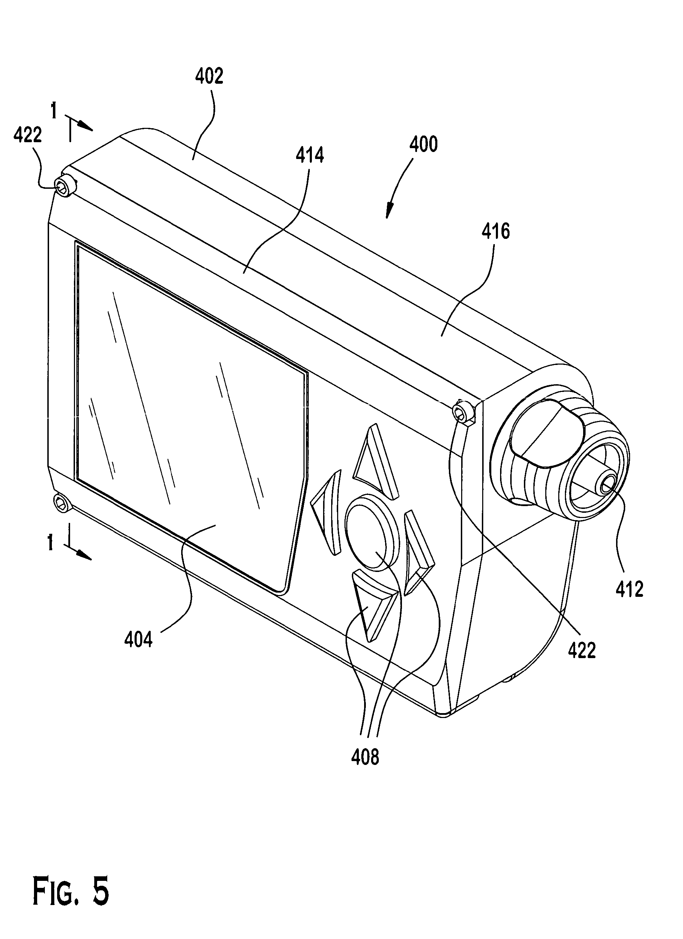

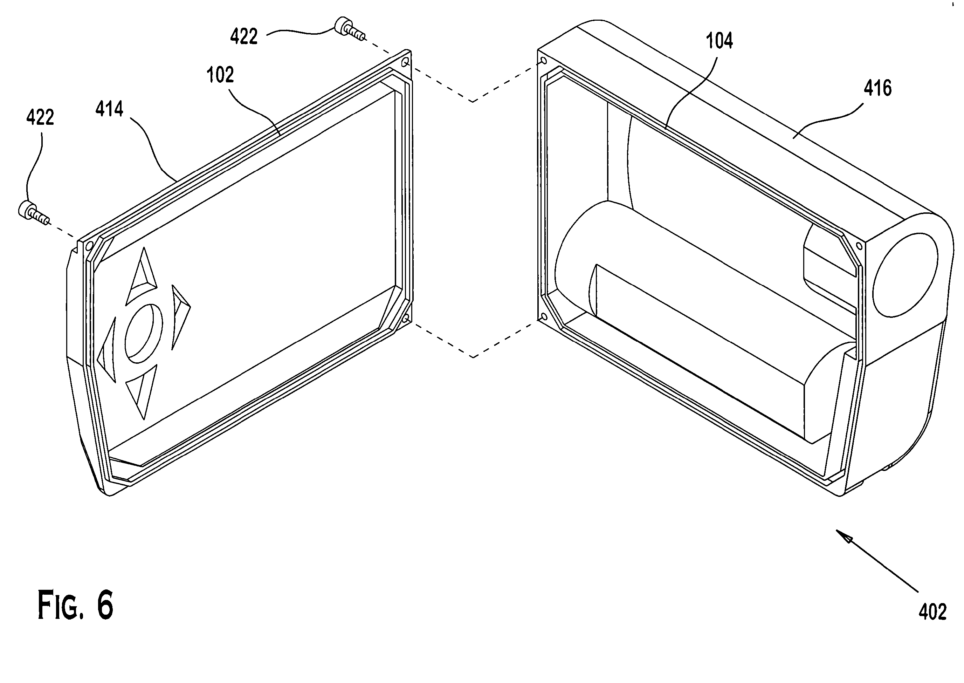

The seal 100 of the present invention may be used in a variety of electrical or electronic devices including, but not limited to, cell phones, telephones, personal data assistants, computers, mini-computers, faxes and hand-held medical devices such as glucose meters and drug delivery devices. An exemplary embodiment of a drug delivery device 400 (e.g., an insulin pump) that may incorporate a seal of the present invention is illustrated in FIGs. 5 and 6. The drug delivery device 400 includes a housing 402, a display 404 for providing operational information to the user, a plurality of navigational buttons 408 for the user to input information, a battery in a compartment (not shown) for providing power to the drug delivery device 400, processing electronics (not shown), a drug delivery mechanism (e.g., an insulin pump and drive mechanism; not shown) for forcing a drug from a cartridge in a chamber through a side port 412 connected to an infusion set (not shown) and into the body of the user.

The housing 402 includes a top portion 414 and a bottom portion 416. A tongue 102 is formed on the periphery of one of the top portion 414 or bottom portion 416 (see FIG. 8). A groove 104 is formed on the periphery of one of the top portion 414 or bottom portion 416 and is configured to receive the tongue 102 such that the housing 402 has at least one portion including a tongue 102 and at least one portion including a groove 104. A coating is formed on at least one surface of the tongue 102 and/or at least one surface of the groove 104 such that the coating creates a hermetic seal 100 between the top portion 414 and the bottom portion 416 when the tongue 102 and the groove 104 are in contact. Injection molding processes such as insert molding, or two-shot over-molding may be used to form the coating on at least one surface of the tongue 102 and/or at least one surface of the groove 104. Use of a coating to form a seal between the tongue 102 and the groove 104 eliminates the need for a separate O-ring.

Referring again to FIG. 6, the top portion 414 includes at least one hole 418 that aligns with at least one hole 420 in the bottom portion 416 through which a fastener 422 such as a bolt fits to hold the top portion 414 and the bottom portion 416 together. The fastener 422 may be held in place by a threaded insert in the bottom portion 416. The fastener 422 allows the easy disassembly of the housing 402 so that the drug delivery device 400 can be serviced.

It will be recognized that equivalent structures may be substituted for the structures illustrated and described herein and that the described embodiment of the invention is not the only structure, which may be employed to implement the claimed invention. In addition, it should be understood that every structure described above has a function and such structure can be referred to as a means for performing that function. While embodiments of the present invention have been shown and described herein, it will be obvious to those skilled in the art that such embodiments are provided by way of example only. Numerous variations, changes, and substitutions will occur to those skilled in the art without departing from the invention.

It should be understood that various alternatives to the embodiments of the invention described herein may be employed in practicing the invention. It is intended that the following claims define the scope of the invention and that methods and structures within the scope of these claims and their equivalents be covered thereby.

### ELEMENTS

| | |
|---|---|
| 100 | seal |
| 102 | tongue |
| 104 | groove |
| 106 | coating |
| 108 | tip |
| 110 | side of tongue |
| 112 | lower surface of groove |
| 114 | at least one wall of groove |
| 200 | seal |
| 206 | coating |
| 300 | seal |
| 306 | coating |
| 400 | drug delivery device |
| 402 | housing |
| 404 | display |
| 406 | keypad |
| 408 | buttons |
| 410 | cap |
| 412 | side port |
| 414 | top portion |
| 416 | bottom portion |
| 418 | hole in top portion |
| 420 | hole in bottom portion |
| 422 | fastener |

## Claims

1. A drug delivery device comprising:
a housing having a top portion and a bottom portion;
a tongue formed on the periphery of one of the top portion or bottom portion;
a groove formed on the periphery of one of the top portion or bottom portion and configured to receive the tongue and such that the housing has at least one portion including a tongue and at least one portion including a groove; and
a coating on at least one surface of the groove that creates a hermetic seal between the top portion and the bottom portion when the tongue and the groove are in contact.

2. A drug delivery device comprising:
a housing having a top portion and a bottom portion;
a tongue formed on the periphery of one of the top portion or bottom portion;
a groove formed on the periphery of one of the top portion or bottom portion and configured to receive the tongue and such that the housing has at least one portion including a tongue and at least one portion including a groove; and
a coating on at least one surface of the tongue that creates a hermetic seal between the top portion and the bottom portion when the tongue and the groove are in contact.

3. A drug delivery device comprising:
a housing having a top portion and a bottom portion;
a tongue formed on the periphery of one of the top portion or bottom portion;
a groove formed on the periphery of one of the top portion or bottom portion and configured to receive the tongue and such that the housing has at least one portion including a tongue and at least one portion including a groove; and
a coating on at least one surface of both the tongue and the groove that creates a hermetic seal between the top portion and the bottom portion when the tongue and the groove are in contact.

4. The drug delivery device of any one of claims 1, 2 or 3, wherein the top portion comprises at least one hole that aligns with at least one hole in the bottom portion through which a fastener fits to hold the top and bottom portions together.

5. A seal between a top portion and a bottom portion of a housing, the seal comprising:
a tongue formed on the periphery of one of the top portion or bottom portion;
a groove formed on the periphery of one of the top portion or bottom portion and configured to receive the tongue and such that the housing has at least one portion including a tongue and at least one portion including a groove; and
a coating on at least one surface of the groove that creates the seal between the top portion and the bottom portion when the tongue and the groove are in contact.

6. A seal between a top portion and a bottom portion of a housing, the seal comprising:
a tongue formed on the periphery of one of the top portion or bottom portion;
a groove formed on the periphery of one of the top portion or bottom portion and configured to receive the tongue and such that the housing has at least one portion including a tongue and at least one portion including a groove; and
a coating on at least one surface of the tongue that creates the seal between the top portion and the bottom portion when the tongue and the groove are in contact.

7. A seal between a top portion and a bottom portion of a housing, the seal comprising:
a tongue formed on the periphery of one of the top portion or bottom portion;
a groove formed on the periphery of one of the top portion or bottom portion and configured to receive the tongue and such that the housing has at least one portion including a tongue and at least one portion including a groove; and
a coating on at least one surface of both the groove and the tongue that creates the seal between the top portion and the bottom portion when the tongue and the groove are in contact.

8. The drug delivery device of any one of claims 1 to 4 or the seal of claim 6 or 7, wherein the coating is a thermoplastic elastomer.

9. The drug delivery device of any one of claims 1 to 4 or the seal of claim 6 or 7, wherein the coating is a copolymer of butadiene and acrylonitrile.

10. The drug delivery device of any one of claims 1 to 4 or the seal of claim 6 or 7, wherein the coating is silicone rubber.

11. The drug delivery device of any one of claims 1 to 4 or the seal of claim 6 or 7, wherein the coating is Sorbothane®.

12. The drug delivery device of anyone of claims 1 to 4 or the seal of claim 6 or 7, wherein the coating is Santoprene®.

13. The drug delivery device of any one of claims 1 to 4 or the seal of claim 6 or 7, wherein the coating is neoprene.
